(19) **Europäisches Patentamt / European Patent Office / Office européen des brevets**

(11) **EP 1 743 178 B1**

(12) **FASCICULE DE BREVET EUROPEEN**

(45) Date de publication et mention
de la délivrance du brevet:
**16.06.2010 Bulletin 2010/24**

(51) Int Cl.:
***G01N 33/68*** *(2006.01)*

(21) Numéro de dépôt: **05762419.9**

(86) Numéro de dépôt international:
**PCT/FR2005/001020**

(22) Date de dépôt: **25.04.2005**

(87) Numéro de publication internationale:
**WO 2005/106481 (10.11.2005 Gazette 2005/45)**

(54) **PROCEDE D'IDENTIFICATION D'UN LIGAND CAPABLE DE MODULER SELECTIVEMENT UNE CASCADE FONCTIONNELLE IMPLIQUANT UNE CIBLE, ET SES APPLICATIONS POUR LE CRIBLAGE A HAUT-DEBIT DE MOLECULES D'INTERET.**

NACHWEISVERFAHREN FÜR EINEN LIGANDEN DER SELEKTIV EINE FUNKTIONELLE KASKADE MODULIEREN KANN UND EIN TARGET IMPLIZIERT SOWIE DESSEN VERWENDUNG FÜR HOCHDURCHSATZ-SCREENING VON MOLEKÜLEN DIE VON INTERESSE SIND

METHOD FOR IDENTIFYING A LIGAND CAPABLE OF SELECTIVELY MODULATING A FUNCTIONAL CASCADE INVOLVING A TARGET, AND USES THEREOF FOR HIGH-THROUGHPUT SCREENING OF MOLECULES OF INTEREST

(84) Etats contractants désignés:
**AT BE BG CH CY CZ DE DK EE ES FI FR GB GR HU IE IS IT LI LT LU MC NL PL PT RO SE SI SK TR**

(30) Priorité: **27.04.2004 FR 0404433**

(43) Date de publication de la demande:
**17.01.2007 Bulletin 2007/03**

(73) Titulaires:
• **Centre National de la Recherche Scientifique**
**75016 Paris (FR)**
• **Université de Montpellier I**
**34006 Montpellier Cedex 1 (FR)**
• **Universite de Montpellier II**
**34095 Montpellier Cedex 5 (FR)**

(72) Inventeurs:
• **MARTINEAU, Pierre**
**F-34980 Saint Gely Du Fesc (FR)**
• **DARIAVACH, Piona**
**F-34090 Montpellier (FR)**

(74) Mandataire: **Almond-Martin, Carol et al**
**Ernest Gutmann - Yves Plasseraud S.A.S.**
**88, Boulevard des Belges**
**69452 Lyon Cedex 06 (FR)**

(56) Documents cités:
WO-A-01/01137        WO-A-96/04557
WO-A-96/04557        WO-A-03/059943
WO-A-2004/020619     WO-A-2004/046192

• **DAUVILLIER S ET AL: "Intracellular single-chain variable fragments directed to the Src homology 2 domains of Syk partially inhibit FcepsilonRI signaling in the RBL-2H3 cell line" JOURNAL OF IMMUNOLOGY, THE WILLIAMS AND WILKINS CO. BALTIMORE, US, vol. 169, no. 5, 1 septembre 2002 (2002-09-01), pages 2274-2283, XP002284889 ISSN: 0022-1767 cité dans la demande**
• **KREBS B ET AL: "High-throughput generation and engineering of recombinant human antibodies" JOURNAL OF IMMUNOLOGICAL METHODS, ELSEVIER SCIENCE PUBLISHERS B.V.,AMSTERDAM, NL, vol. 254, no. 1-2, 1 août 2001 (2001-08-01), pages 67-84, XP004245443 ISSN: 0022-1759**

**Description**

La présente invention est relative à un procédé d'identification d'un ligand capable de moduler sélectivement une cascade fonctionnelle impliquant une molécule-cible ou cible, ainsi qu'à ses applications pour le criblage à haut-débit de molécules d'intérêt, notamment thérapeutique (médicament).

Les avancées récentes dans le domaine de la génomique et de la protéomique, qui ont conduit à l'identification d'un nombre considérable de nouvelles cibles thérapeutique potentielles, ont ouvert des perspectives très intéressantes dans le domaine pharmaceutique.

Toutefois, la découverte de nouveaux médicaments implique de mettre au point des méthodes de criblage à haut-débit de banques de molécules qui soient efficaces, c'est-à-dire permettant d'isoler à partir d'un nombre restreint de molécules sélectionnées (touches ou *hits*), des molécules actives *in vivo* sur la pathologie que l'on cherche à traiter (têtes de série ou *leads*).

En effet, les tests fonctionnels (*in vitro* en culture cellulaire ou *in vivo* dans un modèle animal approprié) qui permettent de vérifier l'activité des touches sélectionnées lors du criblage ne sont généralement pas adaptables au haut-débit.

La plupart des protéines eucaryotes étant multi-fonctionnelles et impliquées dans plusieurs cascades fonctionnelles, les molécules isolées doivent être capables de moduler spécifiquement la propriété d'intérêt de la cible sans affecter ses autres activités dont la modulation pourrait avoir des effets délétères pour la cellule.

En conséquence, le criblage doit être hautement spécifique pour restreindre au maximum le nombre de touches (hits) identifiées, de façon à n'avoir qu'à en tester un faible nombre dans des tests fonctionnels *in vivo*.

Ces méthodes de criblage doivent en outre être faciles à mettre en oeuvre et applicables à toute cible.

Dans ce but, des méthodes de criblage reposant sur l'identification de molécules (mimotopes) mimant l'interaction entre la cible et un peptide ou un oligo- nucléotide (aptamère) ont été proposées.

A titre d'exemple, la demande WO 03/059943 décrit l'identification de peptides se liant à des protéines kinases et pouvant être utilisés pour le criblage de banques de molécules dans des essais compétitifs. La demande WO 01/01137 est relative à des protéines de fusion utilisées pour l'étude des interactions protéine-protéine, dans lesquelles un domaine de liaison modulaire d'une première protéine est remplacé par un anticorps à simple chaine (sFv) et le site de liaison pour un domaine de liaison modulaire sur une deuxième protéine est remplacé par un épitope se liant à cet anticorps scFv. La demande WO 2004/020619 est relative notamment à l'emploi d'anticorps dirigés contre des mutants de la protéine PLC-gamma2 dans des modèles animaux pour l'étude des dysfonctionnements liés à la protéine PLC-gamma2. Enfin, la demande WO 96/04557 décrit l'utilisation d'anticorps recombinants pour identifier les surfaces actives de cibles pharmaceutiques.

Les méthodes de criblage utilisant des aptamères et/ou des peptides sont illustrées dans Lapan et al., Expert Opin. Ther. Targets, 2002, 6, 507-516 ; Green et al., BioTechniques, 2001, 30, 1094-1110 ; Burgstaller et al., Drug Discovery Today, 2002, 7, 1221-1228 ; Demandes Internationales PCT WO 98/19162 et WO 03/059943 et Brevet américain US 6,329,145.

Dans un premier temps, des aptamères/peptides capables de se lier à la cible sont sélectionnés *in vitro* puis leur activité modulatrice, vis-à-vis de la cible est vérifiée par un test fonctionnel *in vitro* dans un système cellulaire approprié ou *in vivo* dans un modèle animal approprié.

Dans un deuxième temps, les aptamères/peptides possédant une activité modulatrice sur la cible sont utilisés dans des tests de liaison compétitive pour identifier des touches (*hits*) capables de déplacer la liaison existante entre l'aptamère/peptide et la cible. Ensuite, l'activité modulatrice des touches vis-à-vis de la cible est vérifiée par un test fonctionnel *in vitro* en culture cellulaire ou *in vivo* dans un modèle animal approprié, de façon à isoler des molécules "têtes de série" (leads).

Ces méthodes ont été validées avec des cibles telles que l'isoforme BB du facteur de croissance humain dérivé des plaquettes (PDGF BB) et la protéine Rev du virus de l'immunodéficience humaine (VIH).

Les Inventeurs se sont donné pour but, la mise au point de méthodes de criblage alternatives. Ils ont montré que la substitution des aptamères ou des peptides modulateurs par des anticorps ou des fragments d'anticorps modulateurs, dans les méthodes de criblage telles que décrites dans l'art antérieur, permettait d'identifier des molécules capables de moduler sélectivement une cascade fonctionnelle impliquant une cible (cascade métabolique, cascade d'activation, cascade de signalisation...).

Les anticorps présentent l'avantage de reconnaître n'importe quelle région d'une protéine, de manière spécifique et avec une affinité élevée (de l'ordre du nM). En particulier, ils peuvent reconnaître les sites de surface de cette protéine et sont donc très intéressants pour inhiber spécifiquement les régions de protéines impliquées dans des cascades fonctionnelles via des interactions protéine-protéine ; il a notamment été montré que des fragments d'anticorps (scFv) dirigés contre les domaines SH2 de la protéine tyrosine kinase Syk, étaient capables d'inhiber spécifiquement *in vivo,* la voie de la phospholipase C-$\gamma$2 (PLC-$\gamma$2 ; Dauvililiers et al., J. Immunol., 2002,169, 2274-2283). En conséquence, les mimotopes isolés par un procédé de criblage mettant en oeuvre des anticorps ou des fragments d'anticorps devraient présenter les mêmes propriétés, notamment en terme affnité/spécificité pour la cible.

Par exemple, les Inventeurs ont criblé une banque de 3000 molécules à l'aide d'un anticorps dirigé contre les domaines SH2 de la protéine tyrosine kinase Syk et capable d'inhiber la voie de la PLCγ-2 ; parmi les 10 ligands de la protéine Syk qu'ils ont sélectionnés, l'un au moins était une molécule inhibitrice *in vivo*, agissant sur la voie de la PLC-γ2, sans altérer celle de Ras également dépendante de la protéine Syk.

Au contraire, il n'a pas été démontré que des aptamères et des peptides étaient capables de reconnaître tout épitope en surface d'un antigène. De part leur petite taille, les peptides et les aptamères, sont plus adaptés pour interagir dans les cavités des cibles plutôt qu'en surface, ce qui est favorable à l'inhibition d'une activité enzymatique mais peu intéressant pour inhiber des cascades fonctionnelles.

Par ailleurs, un certain nombre d'anticorps monoclonaux recombinants sont utilisés dans les traitements thérapeutiques. On peut citer par exemple, le cétuximab (anti-EGFR), le rituximab (anti-CD20), l'infliximab (anti-TNF)... Ces anticorps sont, soit des anticorps chimériques, le plus souvent souris/humain, soit des anticorps humanisés. Ces anticorps sont difficiles à produire et présentent un coût élevé. Le procédé de criblage proposé par les Inventeurs permet de substituer ces anticorps par des molécules chimiques ayant les mêmes propriétés inhibitrices, plus faciles à produire à un coût très largement inférieur (d'au moins un facteur 10).

La présente invention a en conséquence pour objet, un procédé d'identification d'un ligand capable de moduler sélectivement une cascade fonctionnelle impliquant une cible d'une cellule d'un organisme humain, animal ou végétal ou d'un micro-organisme, ladite cascade fonctionnelle impliquant une succession de réactions dans ladite cellule, chacune des réactions étant dépendante des précédentes et aboutissant à une activité biologique mesurable dans ladite cellule, **caractérisé en ce qu**'il comprend au moins les étapes suivantes :

a) l'identification d'un anticorps intracellulaire ou d'un fragment d'anticorps intracellulaire, pouvant être exprimé dans ladite cellule et comprenant au moins l'un des domaines variables d'une chaîne d'immunoglobuline, capable de se lier à ladite cible et de moduler ladite cascade fonctionnelle impliquant ladite cible,

b) le criblage à partir d'une banque de molécules de masse moléculaire inférieure à 2500 Da autres que des oligomères, cycliques ou non cycliques, des ligands modulateurs de la liaison entre ladite cible et l'anticorps ou le fragment d'anticorps identifié en a) , et

c) l'identification à partir des ligands modulateurs obtenus en b), de ceux capables de moduler ladite cascade fonctionnelle.

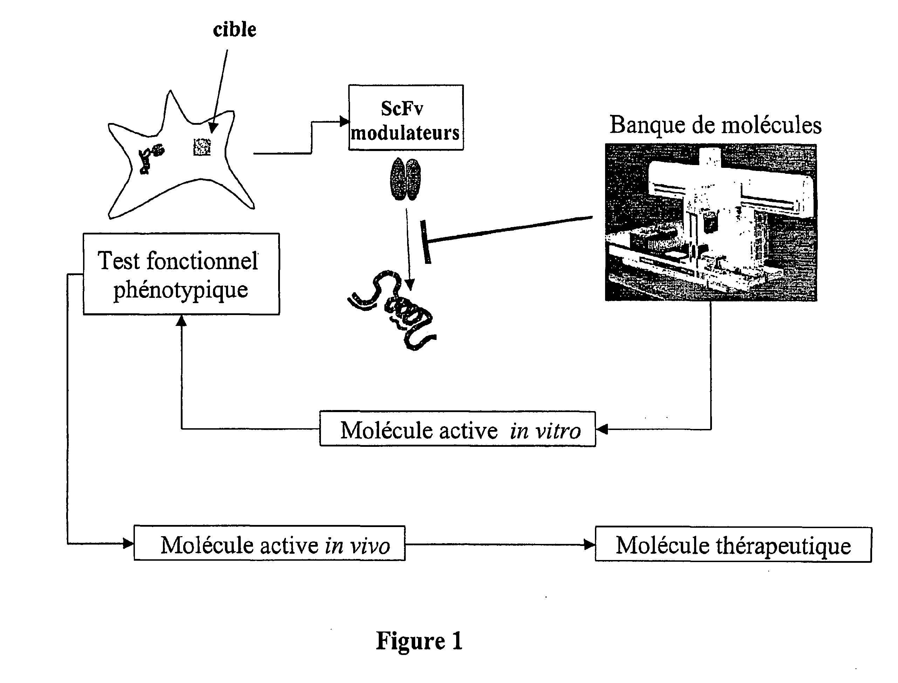

Les étapes du procédé selon l'invention, telles que définies ci-dessus sont schématisées dans la figure 1.

**Définitions**

On entend par :

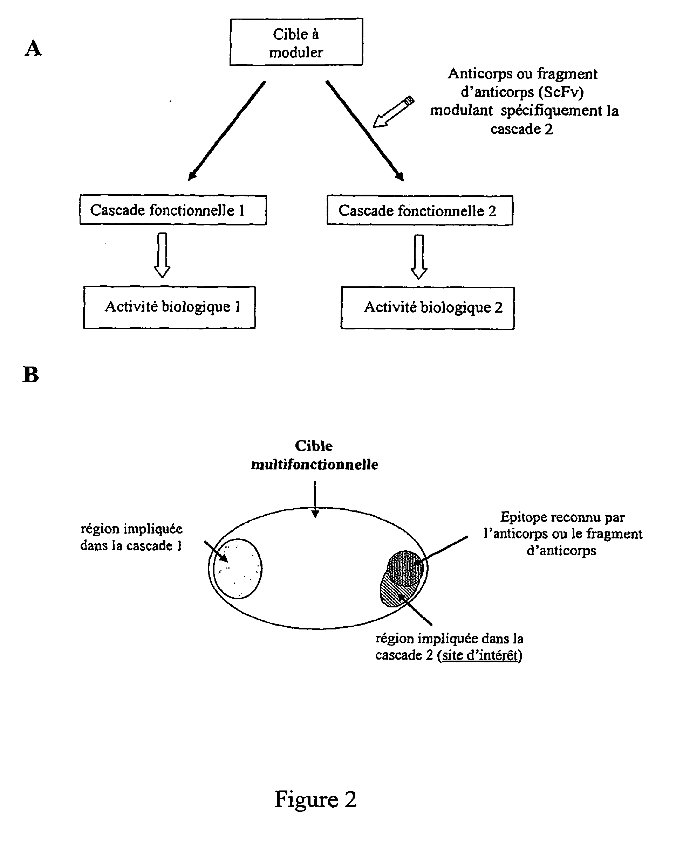

- « cascade fonctionnelle », une voie métabolique, une voie de signalisation ou une cascade d'activation impliquant une succession de réactions (réactions enzymatiques, interactions moléculaires, changements de conformation, modifications chimiques) dans une cellule, chacune des réactions étant dépendante des précédentes et aboutissant à une activité biologique mesurable dans ladite cellule,
- « modulation », une modulation positive ou négative : activation ou inhibition, totale ou partielle,
- « cible impliquée dans une cascade fonctionnelle », toute molécule d'une cellule d'un organisme (humain, animal, végétal) ou d'un micro- organisme (virus, bactérie, champignon, parasite) qui est capable d'interagir avec un ou plusieurs ligands (ou partenaires naturels, connus ou inconnus), chacun se liant à un site ou région distinct de ladite cible (figure 2) ; liaison du ligand module une cascade fonctionnelle impliquée dans un processus physiologique ou pathologique de ladite cellule ou dudit microorganisme et résulte en une activité biologique mesurable dans ladite cellule ou ledit microorganisme. Ladite cible peut être notamment : protéine, un peptide, un polynucléotide (ADN, ARN), un lipide, un sucre ou un dérivé des précédents (glycoprotéine, glycolipide...).

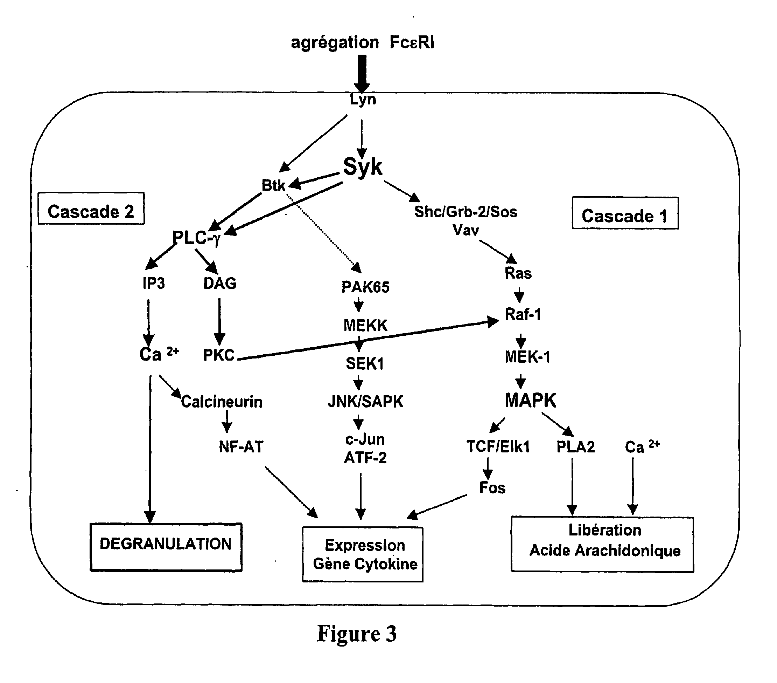

La figure 3 illustre l'exemple de la protéine tyrosine kinase (PTK) Syk qui est une protéine multifonctionnelle impliquée entre autre dans deux cascades d'activation : la première (cascade 1) qui conduit à l'activation de la protéine Ras puis de la MAPK, intervient dans la croissance et la différenciation de certaines cellules de la lignée lymphoïde dont les lymphocytes B, les mastocytes, les basophiles et une sous-population de lymphocytes T (première activité biologique), et la seconde (cascade 2) qui conduit à l'activation de la PTK Btk, de la phospholipase C-γ2 (PLCγ2) et à l'augmentation du flux de calcium intracellulaire, résulte en la dégranulation et la libération de médiateurs allergiques par les mastocytes (seconde activité biologique). La cascade fonctionnelle que l'on cherche à inhiber sélectivement est la cascade 2 qui a des applications dans la prévention et/ou le traitement des allergies, alors que la cascade 1 ne doit pas être inhibée car elle pourrait résulter en des effets secondaires néfastes pour les cellules (agressivité des tumeurs ; Coopman et al.,

Nature, 2000, 406, 742-747).

- « banque de molécules» ou chimiothèque, un ensemble de molécules autres que des anticorps ou des fragments d'anticorps, apparentées par leur structure, leur origine ou leur fonction, notamment une banque combinatoire incluant des molécules qui diffèrent entre elles par le remplacement systématique ou aléatoire de leurs constituants élémentaires, par exemple une banque d'oligomères tels que des peptides, des oligonucléotides (aptamères) et des oligosaccharides ou bien une banque de molécules organiques autres que des oligomères, cycliques ou non-cycliques, notamment des petites molécules organiques, c'est-à-dire de masse moléculaire inférieure à 2500 Da, de préférence inférieure à 2000 Da, de manière préférée inférieure à 1500 Da, de manière plus préférée inférieure à 1000 Da, de manière encore plus préférée inférieure à 750 Da.

- "fragment d'anticorps", un fragment capable de se lier à une cible, comportant au moins le domaine variable d'une chaîne lourde (VH) et/ou le domaine variable d'une chaîne légère (VL) d'une immunoglobuline classique, ou bien le domaine variable d'une immunoglobuline à chaîne unique, tel que les fragments Fab, Fv, scFv ou VHH.

- « anticorps ou fragment d'anticorps capable de se lier à ladite cible et de moduler une cascade fonctionnelle impliquant ladite cible », un anticorps ou un fragment d'anticorps capable de se lier à ladite cible et de mimer l'interaction *in vivo* de ladite cible avec son ligand (partenaire naturel), au niveau du site d'intérêt ; l'épitope de la cible reconnu par ledit anticorps ou fragment d'anticorps est partiellement ou totalement chevauchant avec le site d'intérêt de ladite cible (figure 2B).

Le criblage de la banque de molécules [étape b)] ou d'une banque d'anticorps ou de fragments d'anticorps [étape a) d'identification des anticorps ou fragments d'anticorps capables de se lier à la cible] sont réalisées par toute méthode connue de l'Homme du métier permettant la mesure de l'interaction entre deux partenaires (ELISA, transfert d'énergie par résonance (FRET), polarisation de fluorescence, résonance plasmonique de surface). Les étapes a) et b) sont réalisées à l'aide d'une cible ou d'un dérivé de la cible tel qu'un fragment comprenant au moins le site d'intérêt, un mimotope, ou bien encore un anticorps anti-idiotypique représentant l'image interne dudit site d'intérêt de la cible. La dite cible et ses dérivés ou bien l'anticorps ou le fragment d'anticorps sont éventuellement, immobilisés sur un support approprié, et/ou marqués par tout moyen permettant l'obtention d'un signal mesurable, connus de l'Homme du métier.

L'identification des ligands ou bien des anticorps ou fragments d'anticorps capables de moduler sélectivement une cascade fonctionnelle impliquant ladite cible est réalisée par un test fonctionnel permettant de mesurer l'activité biologique résultant de ladite cascade fonctionnelle. Il s'agit, notamment d'un test *in vitro*, dans un système cellulaire approprié ou *in vivo*, chez un organisme non-human approprié.

Les cellules en culture peuvent être immortalisées ou en culture primaire, adhérentes ou en suspension. L'organisme vivant peut être n'importe quel organisme non humain ou microorganisme de laboratoire ou d'étude (animal (rongeurs, lapins, porcs, bovins, ovins...) ou plante transgénique ou non-transgénique, parasite,..).

Parmi les systèmes cellulaires et les organismes appropriés, on peut citer notamment ceux qui sont des modèles du processus physiologique ou pathologique impliquant ladite cascade fonctionnelle, tels que de façon non-limitative, des modèles du diabète, de l'allergie, de l'arthrite ou de l'asthme.

Avantageusement, l'identification des anticorps ou fragments d'anticorps capables de moduler une cascade fonctionnelle impliquant ladite cible est réalisée dans des cellules modifiées par un vecteur d'expression desdits anticorps ou de leurs fragments (anticorps ou fragments d'anticorps intracellulaires).

Le test fonctionnel mis en jeu dans cette vérification *in vitro,* dans un système cellulaire ou *in vivo* dans un organisme approprié, dépend de l'activité biologique que l'on cherche à moduler dans un but thérapeutique ou non-thérapeutique.

A titre d'exemple non-limitatif d'activité biologique mesurable dans des cellules, on peut citer notamment: la division, la migration, la dégranulation, le transport au travers de membranes, la différenciation, l'apoptose, la réplication, la transcription et la production de facteurs comme des cytokines. Les tests fonctionnels permettant de mesurer ladite activité biologique *in vitro* ou *in vivo* sont connus de l'Homme du métier.

L'identification dudit anticorps ou fragment d'anticorps en a) est réalisée par criblage d'une banque d'anticorps ou de fragments d'anticorps, de préférence une banque de fragments scFv, de manière préférée, une banque de phages exprimant des fragments scFv à leur surface.

L'identification dudit anticorps peut comprendre :

- une première étape de criblage des anticorps ou des fragments d'anticorps capables de se lier à ladite cible, notamment en phage-ELISA et

- une seconde étape d'identification des anticorps ou fragments d'anticorps capables de moduler ladite cascade fonctionnelle, en particulier dans des cellules modifiées par un vecteur d'expression desdits anticorps ou fragments d'anticorps (anticorps ou fragments d'anticorps intracellulaires).

De manière plus précise, les anticorps ou les fragments d'anticorps, notamment les fragments scFv sélectionnés lors de l'étape de criblage peuvent ensuite être clonés dans un vecteur approprié et exprimés dans des cellules, notamment des cellules eucaryotes telles que des cellules de mammifère modifiées par le vecteur recombinant ainsi obtenu. De tels anticorps ou fragments d'anticorps, dénommés anticorps intracellulaires (Cattaneo et al., Trends in Biotechnology, 1999, 17, 115-121), sont exprimés sous forme fonctionnelle, capables de lier la cible dans le compartiment des cellules modifiées dans lequel elle est exprimée (noyau, cytoplasme, compartiment sécrétoire). En outre, des mutations peuvent être introduites dans la séquence des anticorps ou des fragments d'anticorps sélectionnés, de façon à améliorer leur solubilité et/ou leur conformation dans les cellules, notamment chez les eucaryotes. Parmi les mutations envisageables, on peut citer notamment les mutations ponctuelles décrites dans Hurle et al., PNAS USA, 1994, 91, 5446-5450 et Martineau et al., J. Mol. Biol., 1998, 280, 117-127. En outre, des anticorps ou des fragments d'anticorps présentant une stabilité et/ou une affinité augmentée peuvent être sélectionnés par une étape additionnelle de sélection phénotypique, comme décrit dans Cattaneo et al., précité.

Alternativement, l'identification des anticorps ou fragments d'anticorps capables de moduler ladite cascade fonctionnelle est réalisée par une sélection phénotypique appropriée, de cellules modifiées par une banque d'anticorps ou de fragments d'anticorps, selon le principe tel que décrit dans Cattaneo et al., précité.

Selon un autre mode de mise en oeuvre avantageux dudit procédé, l'étape b) de criblage comprend :

- la mise en contact de la banque de molécules à tester avec la cible ou l'un de ses dérivés tels que définis ci-dessus,
- l'addition de l'anticorps ou du fragment d'anticorps, éventuellement marqué, et
- la mesure, par tout moyen approprié, de la quantité relative d'anticorps lié à la cible, en présence ou en l'absence de ladite banque de molécules.

Selon un autre mode de mise en oeuvre avantageux dudit procédé, l'étape b) de criblage est réalisée par un test de liaison de ladite cible, en compétition avec ledit anticorps ou ledit fragment d'anticorps, de façon à isoler des ligands de ladite cible qui déplacent ledit anticorps ou ledit fragment d'anticorps du complexe cible/anticorps ou fragment d'anticorps.

Un exemple de ce mode de mise en oeuvre comprend :

- la mise en contact de l'anticorps ou du fragment d'anticorps, éventuellement marqué, avec la cible ou l'un de ses dérivés tels que définis ci-dessus,
- l'addition de la banque de molécules à tester de façon à détecter les molécules capables de déplacer l'anticorps ou le fragment d'anticorps de son complexe avec le fragment de la cible, et
- la mesure, par tout moyen approprié, de la quantité relative d'anticorps lié à la cible, en présence ou en l'absence de ladite banque de molécules.

Un autre exemple de ce mode de mise en oeuvre comprend :

- le mélange de l'anticorps ou du fragment d'anticorps, éventuellement marqué, avec la banque de molécules à tester,
- la mise en contact du mélange avec la cible ou l'un de ses dérivés tels que définis ci-dessus, et
- la mesure, par tout moyen approprié, de la quantité relative d'anticorps lié à la cible, en présence ou en l'absence de ladite banque de molécules.

La capacité d'une molécule à moduler la liaison entre la cible et l'anticorps est déterminée en comparant la valeur du signal de liaison de l'anticorps, en présence ou en l'absence de chacune des molécules de la banque, testées séparément.

Selon un autre mode de mise en oeuvre avantageux dudit procédé, les étapes a) et c) sont réalisées par un test fonctionnel permettant de mesurer l'activité biologique résultant de ladite cascade fonctionnelle, notamment par un test *in vitro,* dans un système cellulaire approprié ou *in vivo,* chez un organisme non-humain approprié.

Selon un autre mode de mise en oeuvre avantageux dudit procédé, ladite cible est sélectionnée parmi : une enzyme, un récepteur, une protéine adaptatrice, un transporteur, une protéine chaperone, une protéine régulatrice.

A titre d'exemple non-limitatif de ces protéines, on peut citer : des enzymes telles que des protéines tyrosine kinase comme la protéine Syk ; des récepteurs de cytokines comme le récepteur de l'IL-13, de l'EGF ou du TNF, des protéines adaptatrices comme SLP-76 et E6-AP ; des protéines chaperones comme HSP70 et HSP60 ; des protéines régulatrices comme NF-$\kappa$B, I-$\kappa$B, Akt, PSAT-1, p53, p73 et la famille Bcl2.

Selon encore un autre mode de mise en oeuvre avantageux dudit procédé, ledit anticorps ou fragment d'anticorps en a) est dirigé contre les domaines SH2 de la protéine Syk et il est capable d'inhiber la voie de la PLC$\gamma$-2. Un tel anticorps permet d'identifier des médicaments agissant spécifiquement sur les réactions d'hypersensibilité de type I, utiles pour la prévention et/ou le traitement de pathologies telles que la conjonctive ou la rhinite allergique, l'asthme extrinsèque, l'oedème de Quincke et le choc anaphylactique, dans les cas les plus graves.

Selon un autre mode de mise en oeuvre avantageux dudit procédé, ladite banque de molécules en b) est une banque de petites molécules organiques.

La présente invention a également pour objet un kit pour la mise en oeuvre du procédé tel que défini ci-dessus, **caractérisé en ce qu'**il comprend au moins :

- une cible d'une cellule d'un organisme humain, animal ou végétal ou d'un micro-organisme, impliquée dans une cascade fonctionnelle impliquant une succession de réactions dans ladite cellule, chacune des réactions étant dépendante des précédentes et aboutissant à une activité biologique mesurable dans ladite cellule, un fragment de ladite cible comprenant au moins le site d'intérêt, un mimotope de ladite cible, ou bien encore un anticorps anti-idiotypique représentant l'image interne dudit site d'intérêt de la cible tels que définis ci-dessus,
- un anticorps intracellulaire ou un fragment d'anticorps intracellulaire, pouvant être exprimé dans ladite cellule et comprenant au moins l'un des domaines variables d'une chaîne d'immunoglobuline, capable de se lier à ladite cible et de moduler ladite cascade fonctionnelle impliquant ladite cible ou une banque d'anticorps ou de fragments d'anticorps, tels que définis ci-dessus, et
- une banque de molécules organiques de masse moléculaire inférieure à 2500 Da autres que des oligomères, cycliques ou non cycliques, à tester, telle que définie ci-dessus.

Le procédé selon l'invention peut être utilisé pour cribler n'importe quel type de molécule d'intérêt thérapeutique ou non-thérapeutique, capable d'agir sur un processus physiologique ou pathologique de n'importe quel type de cellule ou de microorganisme tels que définis ci-dessus.

Il peut être utilisé pour cribler de nouvelles familles de molécules présentant de nouvelles propriétés d'intérêt thérapeutique ou non-thérapeutique ou des dérivés de ces molécules présentant une activité spécifique et/ou un index thérapeutique améliorés.

Les banques de molécules sont préparées selon les méthodes classiques de synthèse chimique combinatoire, connues de l'Homme du métier.

L'anticorps ou le fragment d'anticorps tels que définis ci-dessus sont préparés par les techniques classiques connues de l'Homme du métier, telles que celles décrites dans Antibodies : A Laboratory Manual, E. Howell and D Lane, Cold Spring Harbor Laboratory, 1988. De manière plus précise :

- les anticorps monoclonaux sont produits à partir d'hybridomes obtenus par fusion entre des lymphocytes B d'un animal immunisé et des myélomes, selon la technique de Köhler et Milstein (Nature, 1975, 256, 495-497) ; les hybridomes sont cultivés *in vitro,* notamment dans des fermenteurs ou produits *in vivo,* sous forme d'ascite ; alternativement, lesdits anticorps monoclonaux sont produits par génie génétique comme décrit dans Methods in Molecular Biology : Antibody Phage Display Methods and Protoco/s, P.M. O'Brien and R. Aitken. Humana Press, Vol. 178, 2002. Par exemple, des animaux appropriés sont immunisés de façon répétée avec la cible ou l'un de ses fragments, selon un protocole standard comprenant une première immunisation par injection intrapéritonéale de l'antigène dans un volume équivalent d'adjuvant complet de Freund puis une deuxième immunisation (rappel) 15 jours plus tard dans des conditions identiques mais avec cette fois de l'adjuvant incomplet de Freund. Les anticorps monoclonaux sont produits selon un protocole standard comprenant le sacrifice des animaux deux semaines après le dernier rappel, le prélèvement de la rate, la mise en suspension des lymphocytes spléniques et la fusion de ces lymphocytes avec la lignée cellulaire murine SP2/0 qui ne produit aucun anticorps murin, qui est immortalisée, et possède toute la machinerie nécessaire à la sécrétion d'immunoglobulines.
- les banques d'anticorps sont, soit des banques naturelles produites à partir des régions $V_H$ et $V_L$ clonées à partir des ARNm d'hybridomes ou de lymphocytes spléniques d'individus naïfs ou préalablement immunisés, soit des banques recombinantes; par exemple, les fragments Fv, scFv ou Fab sont exprimés à la surface de phages filamenteux selon la technique initialement décrite dans Winter et Milstein, Nature, 1991, 349, 293-299 (Methods in Molecular Biology : Antibody Phage Display Methods and Protocols, P.M. O'Brien and R. Aitken. Humana Press, Vol. 178, 2002 *;* Knappik et al., J. Mol. Biol., 2000, 296, 57-86 ; Vaughan et al., Nature Biotech, 1996, 14,309-314).
- les anticorps spécifiques de la cible sont isolés par criblage des banques telles que définies ci-dessus, notamment des banques de phages ; après plusieurs étapes de sélection, les phages qui expriment les fragments d'anticorps spécifiques de la cible sont isolés et les ADNc correspondant auxdits fragments sont exprimés dans un système d'expression approprié, par les techniques classiques de clonage et d'expression d'ADN recombinant. De préférence, lesdits ADNc sont clonés à la fois dans un vecteur d'expression procaryote et eucaryote, sous forme d'une protéine de fusion comprenant au moins à son extrémité -NH$_2$ ou COOH, une étiquette pour la détection dudit fragment d'anticorps, par exemple un épitope (c-myc, HA). Ladite protéine de fusion peut éventuellement comprendre, à l'une des extrémités, une étiquette pour la purification du fragment d'anticorps, par exemple une séquence polyhistidine pour la purification sur une colonne de nickel-agarose. Ledit vecteur d'expression eucaryote contient, outre les éléments régulateurs de la transcription/traduction appropriés (promoteur, enhancer, séquence consensus de Kozak, signal de polyadénylation....) sous le contrôle desquels sont insérées les séquences codantes telles que définies ci-dessus, les éléments indispensables à l'expression desdits fragments dans le compartiment cellulaire approprié tel que défini ci-dessus, notamment dans le cytoplasme, et éventuellement un marqueur de sélection (gène de résistance à un antibiotique,..) pour la sélection de lignées de cellules, notamment eucaryotes, modifiées de façon stable par ledit vecteur d'expression et produisant les anticorps intracellulaires.

Les anticorps monoclonaux ou leurs fragments tels que définis ci-dessus, sont purifiés par les techniques classiques connues de l'Homme du métier, notamment par chromatographie d'affinité.

La cible ou le fragment comprenant le site d'intérêt peuvent être, soit purifiés à partir de tissus ou de cellules, par les techniques classiques connues de l'Homme du métier, soit produits par les techniques classiques d'ADN recombinant connues de l'Homme du métier, en suivant les protocoles standards tels que ceux décrits notamment dans Current Protocols in Molecular Biology (Frederick M. A USUBEL, 2000, Wiley and son Inc, Library of Congress, USA). Le polynucléotide codant pour ladite protéine ou ledit fragment est cloné dans un vecteur d'expression (plasmide, virus..) dans lequel ledit polynucléotide est placé sous le contrôle d'éléments régulateurs de la transcription et de la traduction appropriés. En outre, ledit vecteur peut comprendre des séquences (étiquettes ou *tag*) fusionnées en phase avec l'extrémité 5' et/ou 3' dudit polynucléotide, utiles pour l'immobilisation, et/ou la détection et/ou la purification de la protéine exprimée à partir dudit vecteur ; ladite protéine est ensuite exprimée dans des cellules hôtes appropriées (bactéries, levures, cellules d'insecte ou de mammifère) et éventuellement purifiée.

Alternativement, le fragment comprenant le site d'intérêt est synthétisé en phase solide, selon la méthode originellement décrite par Merrifield et al. (J. Am. Chem. Soc., 1964, 85: 2149-).

Le marquage de la cible, de l'anticorps ou de son fragment et la détection de la liaison anticorps/cible et ligand/cible, sont réalisés par les techniques classiques connues de l'Homme du métier : (i) par fusion de la séquence codante avec la séquence d'un épitope (c-myc, HA), (ii) par couplage avec un marqueur approprié tel qu'un fluorophore, une enzyme (phosphatase alcaline, peroxydase), un isotope radioactif, ou la biotine, (iii) par un anticorps secondaire marqué ou bien par toute interaction spécifique avec un marqueur approprié. Par exemple, la liaison anticorps/cible et ligand/cible est détectée par transfert de fluorescence entre deux protéines fluorescentes complémentaires (Philipps et al., J. Mol. Biol., 2003, 327, 239), deux fluorophores complémentaires (fluorescéine/téraméthylrhodamine) ou une combinaison des deux. Par exemple, la dite cible ou ledit fragment comprennent un fluorophore accepteur ou donneur respectivement à leur extrémité $NH_2$ et/ou COOH et l'anticorps ou le fragment d'anticorps comprennent le fluorophore complémentaire à leur extrémité $NH_2$ et/ou COOH.

Le procédé selon l'invention présente les avantages suivants :

- il permet d'identifier de nouvelles molécules agissant de façon sélective et spécifique sur un processus physiopathologique, potentiellement utilisables comme médicament, par la combinaison de deux étapes de criblage :

  - une étape de criblage par un test de liaison, aisément automatisable et adaptable au haut débit, des molécules qui miment l'interaction d'une cible avec un anticorps ou un fragment d'anticorps (approche idiotypique) et sont donc susceptibles de moduler la cascade fonctionnelle impliquant ladite cible, et
  - une étape de criblage à bas débit, par un test fonctionnel en culture cellulaire ou dans un modèle animal approprié, des molécules capables de moduler la cascade fonctionnelle impliquant ladite cible, notamment par mesure de l'activité biologique résultant de ladite cascade.

  Par exemple, dans le cas de la protéine tyrosine kinase Syk, les résultats obtenus à partir d'une banque de 3000 molécules montrent que l'efficacité de la première étape de criblage avec l'anticorps anti-Syk est telle que la deuxième étape de criblage fonctionnel sur un nombre restreint de molécules (10 molécules), permet d'isoler au moins une molécule inhibitrice active *in vivo* sur la cascade fonctionnelle sélectionnée.

- dans la mesure où il utilise une approche idiotypique, il présente les avantages suivants à savoir :

  - il est adapté à toutes les cibles cellulaires,
  - il est adapté à la reconnaissance de n'importe quel site d'une cible d'intérêt, de manière spécifique et avec une affinité élevée (de l'ordre du nM) et, notamment un site de la surface de ladite cible, susceptible de lier un partenaire par l'intermédiaire d'une interaction du type protéine-protéine ; en conséquence les ligands isolés par ce procédé de criblage devraient présenter les mêmes propriétés, notamment en terme affinité/spécificité pour la cible,
  - il n'implique pas de connaître le partenaire naturel dont l'interaction avec la cible est mimé par l'anticorps et le ligand sélectionné à partir de la banque de molécules, et

- il permet d'identifier de nouvelles molécules permettant avantageusement de remplacer les anticorps monoclonaux recombinants utilisés dans les traitements thérapeutiques comme le cétuximab (anti-EGFR), le rituximab (anti-CD20) et l'infliximab (anti-TNF) qui sont difficiles à produire et présentent un coût élevé.

Outre les dispositions qui précèdent, l'invention comprend encore d'autres dispositions qui ressortiront de la description qui va suivre, qui se réfère à des exemples de criblage de molécules capables de moduler sélectivement la dégranulation des mastocytes, à l'aide d'un fragment scFv dirigé contre les domaines SH2 de la cible Syk, de la famille des PTK (protéine tyrosine kinase), ainsi qu'aux dessins annexés dans lesquels :

- la figure 1 est une représentation schématique du procédé selon l'invention,
- la figure 2 est une représentation schématique d'une cible et de la cascade fonctionnelle dans laquelle elle est impliquée,
- la figure 3 est une représentation schématique des cascades d'activation impliquant la protéine tyrosine kinase Syk et des activités biologiques résultantes,
- la figure 4 illustre la validation du procédé selon l'invention en utilisant la protéine Syk comme cible et le fragment scFv G4G11 comme anticorps pour le criblage d'une chimiothèque. Parmi les dix molécules issues du criblage en ELISA, au moins l'une d'entre elle inhibe fortement le relargage des médiateurs de l'inflammation dans le test de libération de la béta-hexosaminidase : à la concentration de 3 $\mu$M la drogue inhibe à 93 % la dégranulation induite par l'IgE mais pas celle induite par l'ionomycine,

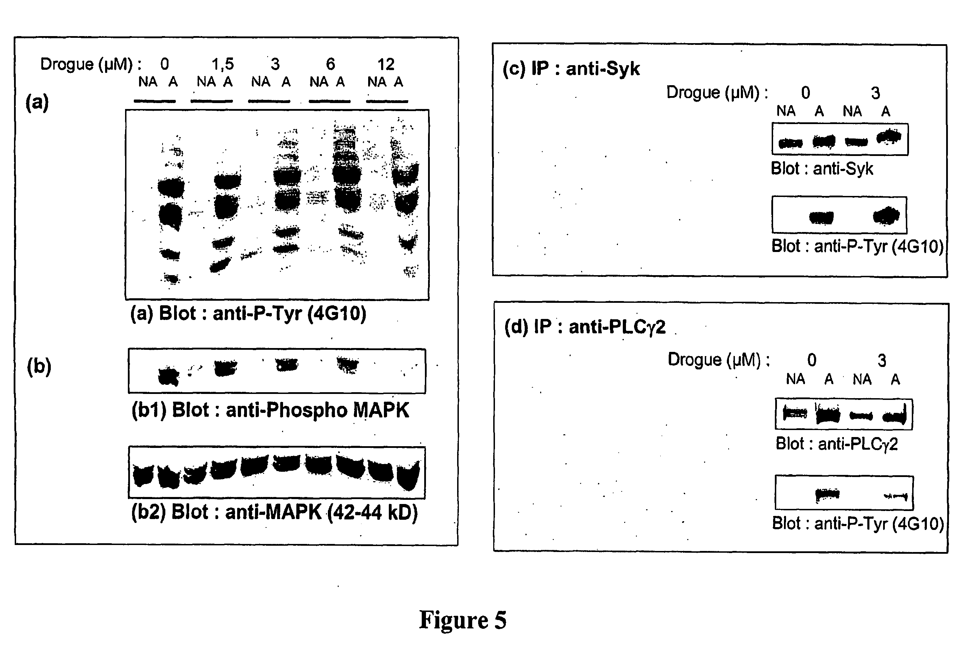

- la figure 5 illustre la comparaison des effets biologiques de la drogue avec l'inhibition observée avec le scFv. Des lysats de cellules RBL-2H3 non activées (NA) et activées (A), en présence de la molécule sélectionnée en ELISA (drogue) à une concentration finale de 1,5 à 12 $\mu$M (a et b) ou de 3 $\mu$M (c et d) ou en l'absence de cette molécule, ont été analysés par : a et b : immunoblot à l'aide d'un anticorps anti-phosphotyrosine 4G10 (a), d'un anticorps anti-phosphoMAPK (b1) ou d'un anticorps anti-MAPK (b2) ; c et d : immunoprécipitation à l'aide d'un anticorps anti-Syk (c) ou anti- PLC-$\gamma$2 (d) puis immunoblot à l'aide du même anticorps utilisé comme contrôle, ou de l'anticorps anti-phosphotyrosine 4G10.

**Exemple : Utilisation d'un fragment scFv dirigé contre les domaines SH2 de la protéine Syk pour cribler des molécules capables d'inhiber la cascade d'activation de la PLC-$\gamma$2 et de la tyrosine kinase Btk qui conduit à la dégranulation des mastocytes et à la libération des médiateurs de l'inflammation.**

La protéine Syk est une protéine tyrosine kinase, multifonctionnelle, qui joue un rôle important dans l'activation de certaines cellules de la lignée lymphoïde dont les lymphocytes B, les mastocytes et basophiles, et une sous-population de lymphocytes T. L'activation de la lignée mastocytaire, suite à la reconnaissance par le récepteur des IgE (FcaRI), d'IgE associées à un allergène, résulte en la libération de médiateurs allergiques tels que l'histamine et la sérotonine.

De manière plus précise, l'agrégation du récepteur Fc$\varepsilon$RI entraîne rapidement la phosphorylation de la protéine Syk et son activation. Syk ainsi activée phosphoryle à son tour ses substrats cytoplasmiques dont la protéine tyrosine kinase Btk et la phospholipase C-$\gamma$2 (PLC-$\gamma$2), toutes deux impliquées dans la cascade d'activation dont résulte la dégranulation des mastocytes et la libération de médiateurs allergiques (voie de la PLC$\gamma$-2 ; figure 3).

La protéine Syk intervient dans une deuxième voie (figure 3) qui conduit à l'activation de la protéine Ras puis de la MAP kinase (MAPK). Il est important de ne pas perturber cette voie car il a été montré qu'elle intervenait dans la croissance et la différenciation des cellules et que la modification de cette deuxième voie pouvait conduire à des phénomènes malins.

Des travaux antérieurs ont permis de montrer que deux scFv interagissant avec les domaines SH2 étaient capables d'inhiber spécifiquement *in vivo* la voie de la PLC$\gamma$-2 (G4G11, G4E4 ; Dauvillier et al., J. Immunol., 2002, 169, 2274-2283). L'anticorps G4G11 a été utilisé pour cribler des petites molécules organiques capables d'inhiber spécifiquement cette voie, *in vivo*.

**1) Criblage d'une banque de molécules en ELISA**

a) Matériel

La protéine Syk recombinante contenant deux domaines SH2 fusionnés en C terminal de la Glutathion-S-transférase (GST) est produite dans *E.coli* et purifiée à comme décrit dans Peneff et al., précité.

Le fragment scFv d'anticorps humain anti-Syk dénommé G4G11, contenant en C-terminal, une étiquette c-myc, pour la détection à l'aide d'un anticorps et une étiquette polyhistidine pour la purification par chromatographie d'affinité, est produit dans *E.coli* et purifié comme décrit dans Peneff et al., précité.

L'anticorps secondaire est l'anticorps monoclonal 9E10 dirigé contre l'épitope c-myc (EQKLISEEDLN), couplé à la peroxydase (Munro et al., Cell, 1986, 46, 291-300).

La banque de molécules à tester est une banque de 3000 petites molécules organiques (ChemBridge Corporation ; http://chembridge.com).

Toutes les dilutions et incubations ont été réalisées dans un tampon PBS /Tween 20 0,1 % / BSA 0, 1 %.

Les lavages ont été réalisés dans un tampon PBS/Tween 20 0,1 %.

Le substrat est le dihydrochlorure de tétraméthylbenzidine à 0,1 mg/ml dans un tampon phosphate citrate pH 5, additionné de $H_2O_2$ (0,03%).

b) <u>Protocole</u>

La protéine Syk recombinante (10 $\mu$g/ml en PBS) a été adsorbée sur une plaque 96 puits (Maxisorp®, NUNC ; 100 $\mu$l soit 1$\mu$g par puits), pendant une nuit à 4°C. Après un premier lavage, les sites non spécifiques de la plaque ont été saturés avec une solution de PBS/BSA 1% à raison de 250 $\mu$l par puit, pendant une nuit à température ambiante. Après lavage de l'excès de solution de saturation, les molécules de la banque (3000 molécules) ont été ajoutées à la concentration de 20 $\mu$M dans un volume de 50 $\mu$l et les plaques ont été incubées pendant 1 heure à température ambiante. Le fragment scFv a ensuite été ajouté à la concentration de 150 nM dans un volume de 50 $\mu$l et les plaques ont été incubées 1 heure à température ambiante. Après un nouveau lavage, 50 $\mu$l d'anticorps secondaire marqué à la peroxydase ont été ajoutés et les plaques ont été incubées 30 minutes à température ambiante. Après un dernier lavage, le substrat de réaction de la peroxydase a été ajouté dans un volume de 100 $\mu$l, puis les plaques ont été incubées pendant 5 minutes et la réaction a été stoppée par addition de 50 $\mu$l de $H_2SO_4$ 2N. La densité optique à 450 mm a ensuite été mesurée à l'aide d'un lecteur automatique pour plaque ELISA.

La banque a été criblée deux fois dans des expériences indépendantes.

c) <u>Résultats</u>

L'inhibition de la liaison entre le fragment de la protéine Syk et le scFv G4G11 par la molécule à tester est mesurée par le rapport I, égal à :

$$\frac{\text{(signal en absence de drogue)} - \text{(signal en présence de drogue)}}{\text{(signal en absence de drogue)}}$$

Les résultats sont les suivants :

- 2990 molécules ne produisent pas d'inhibition (I = 1 $\pm$ 0,2)
- 8 molécules produisent une faible inhibition (0,4 <I< 0,8)
- 2 molécules produisent une forte inhibition (I $\leq$ 0,3)

**2) Vérification du pouvoir modulateur des molécules sélectionnées, *in vivo,* en culture cellulaire**

La vérification *in vivo* du pouvoir modulateur des molécules sélectionnées en ELISA a été effectuée par un test de dégranulation des mastocytes, par mesure du relargage de la β-hexosaminidase comme décrit dans Dauvillier et al., précité.

De plus, l'effet des molécules sélectionnées en ELISA sur la tyrosine phosphorylation des protéines totales, et des protéines Syk, PLCγ-2 et MAPK a été analysé comme décrit dans Dauvillier et al., précité.

Brièvement, les cellules RBL-2H3 (lignée de leucémie basophile de rat) sont ensemencées dans une plaque à 96 puits à raison de $2.10^5$ cellules / puits. Elles sont sensibilisées en présence d'un anticorps monoclonal IgE anti-DNP, pendant une nuit à 37°C. Les cellules sont ensuite incubées 2 heures à 37°C, en présence de différentes concentrations de la molécule sélectionnée en ELISA, puis elles sont activées par l'antigène DNP-BSA, 3 minutes à 37°C. Le surnageant de culture est ensuite récolté et conservé à 4°C (S1). Les cellules adhérentes sont lysées en présence d'un tampon de lyse contenant 0,1% de Triton X-100 et des inhibiteurs de protéases. Les lysats cellulaires sont également récoltés et conservés à 4°C (S2).

Pour calculer la quantité de l'enzyme β-hexosaminidase relarguée, les solutions S1 et S2 sont incubées avec le substrat de l'enzyme : le 4-nitrophényl-2-désoxy-β-D-glucopyranoside, pendant 1h30 à 37°C. La réaction enzymatique est arrêtée par addition d'une solution de glycine pH 3 et la densité optique est lue à 405 nm. La quantité de β-hexosaminidase relarguée correspond à :

$$\frac{\text{Hex S1}}{(\text{Hex S1} + \text{Hex S2})} \times 100$$

Pour chaque concentration, un test de dégranulation par un ionophore de calcium (ionomycine) a été effectué comme contrôle.

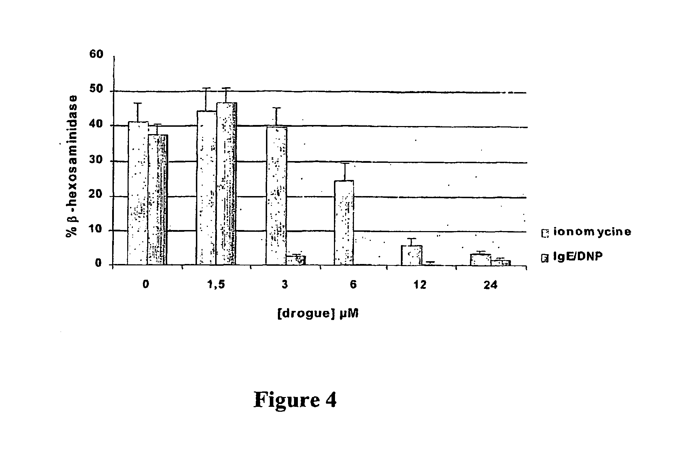

Parmi les dix molécules issues du criblage en ELISA ayant un rapport I ≤ 0,8, l'une d'entre elles inhibe fortement la libération de β-hexosaminidase comme le montre la figure 4. En effet, cette molécule inhibe 93% de la dégranulation induite par l'IgE, à une concentration de 3 μM mais n'affecte pas la dégranulation induite par l'ionomycine.

Des essais complémentaires ont montré que la molécule identifiée se comporte *in vivo* comme le scFv G4G11 et inhibe la voie PLC-γ dépendante, sans affecter la voie Ras dépendante (Figure 5) . En effet :

- elle n'affecte pas la phosphorylation globale des protéines (figure 5a),
- elle n'affecte pas la phosphorylation de Syk (figure 5c),
- elle n'affecte pas la phosphorylation de la MAPK (figure 5b),
- elle diminue la phosphorylation de la PLC-γ (figure 5d).

**3) Vérification du pouvoir modulateur des molécules sélectionnées, *in vivo,* chez la souris.**

La capacité de la molécule la plus active de la figure 4, à inhiber le choc anaphylactique systémique, a été testée chez la souris BALB/c.

De manière plus précise, un groupe de souris (groupe 1) a reçu une une injection d'IgE monoclonale anti-DNP, par voie intra-veineuse, à raison de100 μg par souris BALB/c. Quarante sept heures après, et une heure avant l'injection de l'antigène DNP, la molécule a été administrée par voie orale, à raison de 100 mg/kg, en une seule prise. Une heure après l'administration de la molécule, l'antigène DNP contenant 2% de bleu d'Evans, a été injecté par voie intra-veineuse, à raison de.1 mg par souris. Trois groupes de souris contrôles ont été testés en parallèle avec le groupe 1, à savoir :

- groupe 2 : souris BALB/c ne recevant aucune drogue et permettant de mesurer le choc anaphylactique maximum (contrôle positif).
- groupe 3 : souris BALB/c recevant une molécule inactive.
- groupe 4 : souris recevant l'IgE mais pas l'antigène DNP (contrôle négatif).

L'effet de la molécule sur le choc anaphylactique a ensuite été déterminé par mesure de la libération du bleu d'Evans, par extravasation. Pour ce faire, les souris ont été sacrifiées, puis leurs oreilles ont été coupées. Des surfaces égales des oreilles ont été prélevées, à l'aide d'un emporte-pièce, puis elles ont été hachées, et mises dans une solution de formamide, pendant une nuit à 55°C. Le lendemain, la libération du bleu d'Evans dans la solution de formamide, a été déterminée par mesure de la densité optique à 610 nm.

Les résultats montrent une diminution de 70% de l'extravasation du bleu d'Evans chez les souris ayant reçu la molécule active dans le test d'inhibition de la dégranulation des mastocytes (figure 4), par rapport aux souris contrôles ne recevant aucune molécule ou une molécule inactive. Ces résultats démontrent la capacité de la molécule sélectionnée par le procédé de criblage selon l'invention, à protéger un animal du choc anaphylactique.

**Revendications**

**1.** Procédé d'identification d'un ligand capable de moduler sélectivement une cascade fonctionnelle impliquant une cible d'une cellule d'un organisme humain, animal ou végétal ou d'un micro-organisme, ladite cascade fonctionnelle impliquant une succession de réactions dans ladite cellule, chacune des réactions étant dépendante des précédentes et aboutissant à une activité biologique mesurable dans ladite cellule, comprenant au moins les étapes suivantes :

a) l'identification d'un anticorps intracellulaire ou d'un fragment d'anticorps intracellulaire, pouvant être exprimé dans ladite cellule et comprenant au moins l'un des domaines variables d'une chaîne d'immunoglobuline, capable de se lier à ladite cible et de moduler ladite cascade fonctionnelle impliquant ladite cible,
b) le criblage à partir d'une banque de molécules organiques de masse moléculaire inférieure à 2500 Da autres que des oligomères, cycliques ou non cycliques, des ligands modulateurs de la liaison entre ladite cible et l'anticorps ou le fragment d'anticorps identifié en a) , et

c) l'identification à partir des ligands modulateurs obtenus en b), de ceux capables de moduler ladite cascade fonctionnelle.

**2.** Procédé selon la revendication 1, **caractérisé en ce que** les étapes a) et b) sont réalisées à l'aide d'une cible ou d'un dérivé de la cible tel qu'un fragment comprenant au moins le site d'intérêt, un mimotope, ou bien encore un anticorps anti-idiotypique représentant l'image interne dudit site d'intérêt de la cible.

**3.** Procédé selon la revendication 2, **caractérisé en ce que** la dite cible ou son dérivé ou bien l'anticorps ou le fragment d'anticorps est marqué par tout moyen permettant l'obtention d'un signal mesurable.

**4.** Procédé selon l'une quelconque des revendications 1 à 3, **caractérisé en ce que** l'identification dudit anticorps ou fragment d'anticorps en a) est réalisée par criblage d'une banque de fragments scFv, de manière préférée, une banque de phages exprimant des fragments scFv à leur surface.

**5.** Procédé selon la revendication 4, **caractérisé en ce que** l'identification dudit anticorps ou fragment d'anticorps intracellulaire en a) comprend :

(i) une première étape de criblage des anticorps ou fragments d'anticorps intracellulaires capables de se lier à ladite cible, et
(ii) une seconde étape d'identification des anticorps ou fragments d'anticorps intracellulaires capables de moduler ladite cascade fonctionnelle dans des cellules modifiées par un vecteur d'expression desdits anticorps ou fragments d'anticorps

**6.** Procédé selon l'une quelconque des revendications 1 à 5, **caractérisé en ce que** l'étape b) de criblage comprend :

- la mise en contact de la banque de molécules à tester avec la cible ou l'un de ses dérivés tels que définis à la revendication 2,
- l'addition de l'anticorps ou du fragment d'anticorps, éventuellement marqué, et
- la mesure, par tout moyen approprié de la quantité relative d'anticorps lié à la cible, en présence ou en l'absence de ladite banque de molécule.

**7.** Procédé selon l'une quelconque des revendications 1 à 5, **caractérisé en ce que** l'étape b) de criblage comprend :

- la mise en contact de l'anticorps ou du fragment d'anticorps, éventuellement marqué, avec la cible ou l'un de ses dérivés tels que définis à la revendication 2,
- l'addition de la banque de molécules à tester de façon à détecter les molécules capables de déplacer l'anticorps ou le fragment d'anticorps de son complexe avec le fragment de la cible, et
- la mesure, par tout moyen approprié, de la quantité relative d'anticorps lié à la cible, en présence ou en l'absence de ladite banque de molécule.

**8.** Procédé selon l'une quelconque des revendications 1 à 5, **caractérisé en ce que** l'étape b) de criblage comprend :

- le mélange de l'anticorps ou du fragment d'anticorps, éventuellement marqué, avec la banque de molécules à tester,
- la mise en contact du mélange avec la cible ou l'un de ses dérivés tels que définis à la revendication 2, et
- la mesure, par tout moyen approprié, de la quantité relative d'anticorps lié à la cible, en présence ou en l'absence de ladite banque de molécules.

**9.** Procédé selon l'une quelconque des revendications 1 à 8, **caractérisé en ce que** les étapes

a) et c) sont réalisées par un test fonctionnel permettant de mesurer l'activité biologique résultant de ladite cascade fonctionnelle, notamment par un test in vitro, dans un système cellulaire approprié ou in vivo, chez un organisme non humain approprié.

**10.** Procédé selon l'une quelconque des revendications 1 à 9, **caractérisé en ce** ladite cible est sélectionnée dans le groupe constitué par : une enzyme, un récepteur, une protéine adaptatrice, un transporteur, une protéine chaperone et une protéine régulatrice.

**11.** Procédé selon l'une quelconque des revendications 1 à 10, **caractérisé en ce que** ledit anticorps ou fragment d'anticorps identifié en a) est dirigé contre les domaines SH2 de la protéine Syk et capable d'inhiber spécifiquement la voie de la PLCγ-2.

**12.** Procédé selon l'une quelconque des revendications 1 à 11, **caractérisé en ce que** lesdites petites molécules organiques autres que des oligomères ont une masse moléculaire inférieure à 1000 Da.

**13.** Procédé selon la revendication 12, **caractérisé en ce que** lesdites petites molécules organiques autres que des oligomères ont une masse moléculaire inférieure à 750 Da.

**14.** Procédé selon la revendication 4 ou 5, **caractérisé en ce que** ledit anticorps ou fragment d'anticorps intracellulaire est capable de lier la cible dans le compartiment des cellules modifiées dans lequel elle est exprimée.

**15.** Procédé selon la revendication 14, **caractérisé en ce que** ladite cellule est une cellule eucaryote et plus particulièrement une cellule de mammifère.

**16.** Procédé selon l'une quelconque des revendications 1 à 15, **caractérisé en ce que** la cible est une molécule d'un organisme humain, animal ou végétal.

**17.** Kit pour la mise en oeuvre du procédé selon l'une quelconque des revendications 1 à 16, **caractérisé en ce qu'**il comprend au moins :

- une cible d'une cellule d'un organisme humain, animal ou végétal ou d'un micro-organisme, impliquée dans une cascade fonctionnelle impliquant une succession de réactions dans ladite cellule, chacune des réactions étant dépendante des précédentes et aboutissant à une activité biologique mesurable dans ladite cellule, un fragment de ladite cible comprenant au moins le site d'intérêt, un mimotope de ladite cible, ou bien encore un anticorps anti-idiotypique représentant l'image interne dudit site d'intérêt de la cible,
- un anticorps intracellulaire ou un fragment d'anticorps intracellulaire, pouvant être exprimé dans ladite cellule et comprenant au moins l'un des domaines variables d'une chaîne d'immunoglobuline, capable de se lier à ladite cible et de moduler ladite cascade fonctionnelle impliquant ladite cible, ou bien une banque d'anticorps intracellulaires ou de fragments d'anticorps intracellulaires, pouvant être exprimés dans ladite cellule, et
- une banque de molécules organiques de masse moléculaire inférieure à 2500 Da à tester, autres que des oligomères, cycliques ou non cycliques.

**Claims**

**1.** A method for identifying a ligand capable of selectively modulating a functional cascade involving a target from a cell of a human, animal or plant organism or of a micro-organism, said functional cascade involving a succession of reactions in said cell, each of the reactions being dependent on the preceding ones and resulting in a measurable biological activity in said cell, comprising at least the following steps:

a) identifying an intracellular antibody or an intracellular antibody fragment which can be expressed in said cell and comprising at least one of the variable domains of an immunoglobulin chain, capable of binding to said target and of modulating said functional cascade involving said target,
b) screening, using a library of cyclic or non cyclic organic molecules other than oligomers, having a molecular mass of less than 2500 Da, for ligands that modulate the binding between said target and the antibody or the antibody fragment identified in a), and
c) identifying, from the modulatory ligands obtained in b), those capable of modulating said functional cascade.

**2.** The method according to claim 1, wherein steps a) and b) are carried out using a target or a derivative of the target such as a fragment comprising at least the site of interest, a mimotope, or else an anti-idiotype antibody representing the internal image of said site of interest of the target.

**3.** The method according to claim 2, wherein said target or its derivatives or else the antibody or the antibody fragment is labelled by any means for obtaining a measurable signal.

**4.** The method according to any one of claims 1 to 3, wherein the identification of said antibody or antibody fragment in a) is carried out by screening a library of scFv fragments, preferably a library of phages expressing scFv fragments at their surface.

5. The method according to claim 4, wherein the identification of said antibody or antibody fragment in a) comprises :

(i) a first step consisting in screening intracellular antibodies or antibody fragments capable of binding to said target, and
(ii) a second step consisting in identifying the intracellular antibodies or antibody fragments capable of modulating said functional cascade in cells modified with a vector for expression of said antibodies or antibody fragments.

6. The method according to any one of claims 1 to 5, wherein the screening step b) comprises:

- bringing the library of molecules to be tested into contact with the target or one of its derivatives as defined in claim 2,
- adding the antibody or the antibody fragment, optionally labelled, and
- measuring, by any appropriate means, the relative amount of antibody bound to the target, in the presence or absence of said library of molecules.

7. The method according to any one of claims 1 to 5, wherein the screening step b) comprises:

- bringing the optionally labelled antibody or antibody fragment into contact with the target or one of its derivatives as defined in claim 2,
- adding the library of molecules to be tested so as to detect the molecules capable of displacing the antibody or the antibody fragment from its complex with the fragment of the target, and
- measuring, by any appropriate means, the relative amount of antibody bound to the target, in the presence or absence of said library of molecules.

8. The method according to any one of claims 1 to 5, wherein the screening step b) comprises:

- mixing the optionally labelled antibody or antibody fragment with the library of molecules to be tested,
- bringing the mixture into contact with the target or one of its derivatives as defined in claim 2, and
- measuring, by any appropriate means, the relative amount of antibody bound to the target, in the presence or absence of said library of molecules.

9. The method according to any one of claims 1 to 8, wherein steps a) and c) are carried out by means of a functional test for measuring the biological activity resulting from said functional cascade, in particular by means of an in vitro test, in an appropriate cell system, or an in vivo test, in an appropriate non-human organism.

10. The method according to any one of claims 1 to 9, wherein said target is selected from the group consisting of: an enzyme, a receptor, an adaptor protein, a transporter, a chaperone protein and a regulatory protein.

11. The method according to any one of claims 1 to 10, wherein said antibody or antibody fragment identified in a) is directed against the SH2 domains of the Syk protein and is capable of specifically inhibiting the PLCγ-2 pathway.

12. The method according to any one of claims 1 to 11, wherein said small organic molecules other than oligomers have a molecular mass of less than 1000 Da.

13. The method according to claim 12, wherein said small organic molecules other than oligomers have a molecular mass of less than 750 Da.

14. The method according to claim 4 or 5, wherein said intracellular antibody or antibody fragment is capable of binding to the target in the compartment of the modified cells in which it is expressed.

15. The method according to claim 14, wherein said cell is a eukaryotic cell, and more particularly a mammalian cell.

16. The method according to any one of claim 1 to 15, wherein said target is a molecule from a human, animal or plant.

17. A kit for carrying out the method according to any one of claims 1 to 16, said kit comprising at least:

- a target from a cell of a human, animal or plant organism or of a micro-organism, involved in a functional cascade involving a succession of reactions in said cell, each of the reactions being dependent on the preceding ones and resulting in a measurable biological activity in said cell, a fragment of said target comprising at least the site of interest, a mimotope of said target, or else an anti-idiotype antibody representing the internal image of said site of interest of the target,

- an intracellular antibody or an intracellular antibody fragment which can be expressed in said cell and comprising at least one of the variable domains of an immunoglobulin chain, capable of binding to said target and of modulating said functional cascade involving said target, or else a library of intracellular antibodies or of intracellular antibody fragments which can be expressed in said cell, and

- a library of cyclic or non cyclic organic molecules other than oligomers having a molecular mass of less than 2500 Da, to be tested.

**Patentansprüche**

1. Nachweisverfahren für einen Liganden, der in der Lage ist, selektiv eine funktionelle Kaskade zu modulieren, die ein Target einer Zelle eines menschlichen, tierischen oder pflanzlichen Organismus oder eines Mikroorganismus impliziert, wobei die funktionelle Kaskade eine Abfolge von Reaktionen in der Zelle impliziert, wobei jede der Reaktionen von den vorhergehenden abhängt und zu einer messbaren biologischen Aktivität in der Zelle führt, welches wenigstens die folgenden Schritte aufweist:

   a) das Nachweisen eines intrazellulären Antikörpers oder eines intrazellulären Antikörper-Fragments, der/das in der Zelle exprimiert werden kann und wenigstens eine der variablen Domänen einer Immunoglobulinkette umfasst, die in der Lage ist, an das Target zu binden und die funktionelle Kaskade, die das Target impliziert, modulieren kann,

   b) das Screenen anhand einer Bank organischer Moleküle mit einer Molmasse von weniger als 2500 Da, die keine Oligomere sind, zyklisch oder nicht zyklisch, von Modulator-Liganden der Bindung zwischen dem Target und dem Antikörper oder Antikörper-Fragment wie in a) definiert und

   c) das Nachweisen anhand der in b) erhaltenen Modulator-Liganden jener, die in der Lage sind, die funktionelle Kaskade zu modulieren.

2. Verfahren gemäß Anspruch 1, **dadurch gekennzeichnet, dass** die Schritte a) und b) mit Hilfe eines Targets oder eines Target-Derivats wie einem Fragment, das wenigstens die Stelle von Interesse enthält, einem Mimotop oder auch einem anti-idiotypischen Antikörper, der das innere Abbild der Stelle von Interesse des Targets darstellt, durchgeführt werden.

3. Verfahren gemäß Anspruch 2, **dadurch gekennzeichnet, dass** das Target oder dessen Derivat oder auch der Antikörper oder das Antikörper-Fragment mit jedem beliebigen Mittel markiert ist, welches es ermöglicht, ein messbares Signal zu erhalten.

4. Verfahren gemäß einem der Ansprüche 1 bis 3, **dadurch gekennzeichnet, dass** das Nachweisen des Antikörpers oder des Antikörper-Fragments in a) durch Screenen einer Fragmentbank scFv, vorzugsweise einer Phagenbank, die die Fragmente scFv auf ihrer Oberfläche exprimiert, durchgeführt wird.

5. Verfahren gemäß Anspruch 4, **dadurch gekennzeichnet, dass** das Nachweisen des intrazellulären Antikörpers oder Antikörper-Fragments in a) aufweist:

   (i) einen ersten Schritt des Screenens des intrazellulären Antikörpers oder Antikörper-Fragments, der/das in der Lage ist, an das Target zu binden, und

   (ii) einen zweiten Schritt des Nachweisens des intrazellulären Antikörpers oder Antikörper-Fragments, das in der Lage ist, die funktionelle Kaskade in den Zellen zu modulieren, die durch einen Expressionsvektor der Antikörper oder Antikörper-Fragmentemodifiziert wurden.

6. Verfahren gemäß einem der Ansprüche 1 bis 5, **dadurch gekennzeichnet, dass** der Schritt b) des Screenens aufweist:

   - das Inkontaktbringen der zu testenden Molekülbank mit dem Target oder einem seiner Derivate wie in Anspruch 2 definiert,

- das Hinzufügen des Antikörpers oder Antikörper-Fragments, gegebenenfalls markiert, und
- das Messen mit jedem geeigneten Mittel der relativen Menge an mit dem Target verbundenen Antikörpern, bei Vorhandensein oder Fehlen der Molekülbank.

7. Verfahren gemäß einem der Ansprüche 1 bis 5, **dadurch gekennzeichnet, dass** der Schritt b) des Screenens aufweist:

- das Inkontaktbringen des Antikörpers oder Antikörper-Fragments, gegebenenfalls markiert, mit dem Target oder einem seiner Derivate wie in Anspruch 2 definiert,
- das Hinzufügen der zu testenden Molekülbank, um die Moleküle zu detektieren, die in der Lage sind, den Antikörper oder das Antikörper-Fragment aus seinem Komplex mit dem Fragment des Targets zu versetzen, und
- das Messen mit jedem geeigneten Mittel der relativen Menge an mit dem Target verbundenen Antikörpern, bei Vorhandensein oder Fehlen der Molekülbank.

8. Verfahren gemäß einem der Ansprüche 1 bis 5, **dadurch gekennzeichnet, dass** der Schritt b) des Screenens aufweist:

- das Mischen des Antikörpers oder Antikörper-Fragments, gegebenenfalls markiert, mit der zu testenden Molekülbank,
- das Inkontaktbringen des Gemischs mit dem Target oder einem seiner Derivate wie in Anspruch 2 definiert und
- das Messen mit jedem geeigneten Mittel der relativen Menge an mit dem Target verbundenen Antikörpern, bei Vorhandensein oder Fehlen der Molekülbank.

9. Verfahren gemäß einem der Ansprüche 1 bis 8, **dadurch gekennzeichnet, dass** die Schritte

a) und c) durch einen funktionellen Test durchgeführt werden, der es ermöglicht, die biologische Aktivität zu messen, die sich aus der funktionellen Kaskade ergibt, insbesondere durch einen in vitro-Test in einem geeigneten Zellsystem oder in vivo bei einem geeigneten nicht menschlichen Organismus.

10. Verfahren gemäß einem der Ansprüche 1 bis 9, **dadurch gekennzeichnet, dass** das Target ausgewählt ist aus einer Gruppe bestehend aus: einem Enzym, einem Rezeptor, einem Adapterprotein, einem Transporter, einem Chaperonprotein und einem Regulatorprotein.

11. Verfahren gemäß einem der Ansprüche 1 bis 10, **dadurch gekennzeichnet, dass** der in a) nachgewiesene Antikörper oder das Antikörper-Fragment gegen die Domänen SH2 des Proteins Syk gerichtet ist und in der Lage ist, spezifisch den Weg von PLCy-2 zu hemmen.

12. Verfahren gemäß einem der Ansprüche 1 bis 11, **dadurch gekennzeichnet, dass** die kleinen organischen Moleküle, die keine Oligomere sind, eine Mol-masse von weniger als 1000 Da aufweisen.

13. Verfahren gemäß Anspruch 12, **dadurch gekennzeichnet, dass** die kleinen organischen Moleküle, die keine Oligomere sind, eine Molekularmasse von weniger als 750 Da aufweisen.

14. Verfahren gemäß Anspruch 4 oder 5, **dadurch gekennzeichnet, dass** der intrazelluläre Antikörper oder das Antikörper-Fragment in der Lage ist, das Target in dem modifizierten Zellkompartiment zu binden, in dem es exprimiert ist.

15. Verfahren gemäß Anspruch 14, **dadurch gekennzeichnet, dass** die Zelle eine eukaryotische Zelle ist, und insbesondere eine Säugetierzelle.

16. Verfahren gemäß einem der Ansprüche 1 bis 15, **dadurch gekennzeichnet, dass** das Target ein Molekül eines menschlichen, tierischen oder pflanzlichen Organismus ist.

17. Kit für die Durchführung des Verfahrens gemäß einem der Ansprüche 1 bis 16, **dadurch gekennzeichnet, dass** es wenigstens aufweist:

- ein Target einer Zelle eines menschlichen, tierischen oder pflanzlichen Organismus oder eines Mikroorganismus, der in der funktionellen Kaskade impliziert ist, die eine Abfolge von Reaktionen in der Zelle impliziert, wobei jede der Reaktionen von den vorhergehenden abhängt und zu einer messbaren biologischen Aktivität in der Zelle führt, ein Fragment des Targets, das wenigstend die Stelle von Interesse enthält, ein Mimotop des Targets oder auch einen anti-idiotypischen Antikörper, der das innere Abbild der Stelle von Interesse des Targets darstellt,

- einen intrazellulären Antikörper oder ein intrazelluläres Antikörper-Fragment, der/das in der Zelle exprimiert werden kann und wenigstens eine der variablen Domänen einer Immunoglobulinkette umfasst, die in der Lage ist, an das Target zu binden, und die funktionelle Kaskade, die das Target impliziert, modulieren kann, oder auch eine Bank intrazellulärer Antikörpern oder intrazellulärer Antikörper-Fragmenten, die in der Zelle exprimiert werden können, und

- eine zu testende Bank organischer Moleküle mit einer Molmasse von weniger als 2500 Da, die keine Oligomere sind, zyklisch oder nicht zyklisch.

**Figure 1**

**A**

Cible à moduler

Anticorps ou fragment d'anticorps (ScFv) modulant spécifiquement la cascade 2

Cascade fonctionnelle 1

Cascade fonctionnelle 2

Activité biologique 1

Activité biologique 2

**B**

**Cible multifonctionnelle**

région impliquée dans la cascade 1

Epitope reconnu par l'anticorps ou le fragment d'anticorps

région impliquée dans la cascade 2 (site d'intérêt)

Figure 2

Figure 3

**Figure 4**

**(a)**

Drogue (µM) : 0    1,5    3    6    12
NA A   NA A   NA A   NA A   NA A

**(a) Blot : anti-P-Tyr (4G10)**

**(b)**

**(b1) Blot : anti-Phospho MAPK**

**(b2) Blot : anti-MAPK (42-44 kD)**

**(c) IP : anti-Syk**

Drogue (µM) :   0     3
NA   A   NA   A

Blot : anti-Syk

Blot : anti-P-Tyr (4G10)

**(d) IP : anti-PLCγ2**

Drogue (µM) :   0     3
NA   A   NA   A

Blot : anti-PLCγ2

Blot : anti-P-Tyr (4G10)

**Figure 5**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 03059943 A
- WO 0101137 A
- WO 2004020619 A
- WO 9604557 A
- WO 9819162 A
- US 6329145 B

**Littérature non-brevet citée dans la description**

- **Lapan et al.** *Expert Opin. Ther. Targets,* 2002, vol. 6, 507-516
- **Green et al.** *BioTechniques,* 2001, vol. 30, 1094-1110
- **Burgstaller et al.** *Drug Discovery Today,* 2002, vol. 7, 1221-1228
- **Dauvililiers et al.** *J. Immunol.,* 2002, vol. 169, 2274-2283
- **Coopman et al.** *Nature,* 2000, vol. 406, 742-747
- **Cattaneo et al.** *Trends in Biotechnology,* 1999, vol. 17, 115-121
- **Hurle et al.** *PNAS USA,* 1994, vol. 91, 5446-5450
- **Martineau et al.** *J. Mol. Biol.,* 1998, vol. 280, 117-127
- **E. Howell ; D Lane.** Antibodies : A Laboratory Manual. Cold Spring Harbor Laboratory, 1988
- **Köhler ; Milstein.** *Nature,* 1975, vol. 256, 495-497
- **P.M. O'Brien ; R. Aitken.** Methods in Molecular Biology : Antibody Phage Display Methods and Protoco/s. Humana Press, 2002, vol. 178
- **Winter ; Milstein.** *Nature,* 1991, vol. 349, 293-299
- **P.M. O'Brien ; R. Aitken.** Methods in Molecular Biology : Antibody Phage Display Methods and Protocols. Humana Press, 2002, vol. 178
- **Knappik et al.** *J. Mol. Biol.,* 2000, vol. 296, 57-86
- **Vaughan et al.** *Nature Biotech,* 1996, vol. 14, 309-314
- **Frederick M.** Current Protocols in Molecular Biology. Wiley and son Inc, 2000
- **Merrifield et al.** *J. Am. Chem. Soc.,* 1964, vol. 85, 2149
- **Philipps et al.** *J. Mol. Biol.,* 2003, vol. 327, 239
- **Dauvillier et al.** *J. Immunol.,* 2002, vol. 169, 2274-2283
- **Munro et al.** *Cell,* 1986, vol. 46, 291-300